# EUROPEAN PATENT SPECIFICATION

(11) **EP 3 468 620 B1**
(45) Date of publication and mention of the grant of the patent: **05.05.2021**
(21) Application number: 17776268.9
(22) Date of filing: 07.03.2017
(51) Int. Cl.: A61K 51/04, C07B 59/00, C07F 9/38

(54) **18 F-LABELED BISPHOSPHONATES FOR PET IMAGING**
18-F-MARKIERTE BISPHOSPHONATE ZUR PET-BILDGEBUNG
BISPHOSPHONATES MARQUÉS AU 18F POUR L'IMAGERIE TEP

(30) Priority: 07.03.2016 US 201662304895 P; 06.06.2016 US 201662346391 P
(43) Date of publication of application: 17.04.2019
(73) Proprietor: University of Southern California, Los Angeles, CA 90015 (US)
(72) Inventor: MCKENNA, Charles, E., Pacific Palisades CA 90272 (US); KASHEMIROV, Boris, A., Los Angeles CA 90066 (US); NEGAHBANI, Amirsoheil, Overland Park, Kansas 66213 (US); CHEN, Kai, San Gabriel, CA 91776 (US)
(74) Representative: Schneiders & Behrendt Bochum
(86) International application number: PCT/US2017/021224
(87) International publication number: WO 2017/172303

(56) References cited:
- US-A- 3 627 842
- CHARLES E. MCKENNA ET AL: "Fluorination of methanediphosphonate esters by perchloryl fluoride. Synthesis of fluoromethanediphosphonic acid and difluoromethanediphosphonic acid", J. ORG. CHEM. CARBAZOLE, vol. 46, no. 22, 1 October 1981 (1981-10-01), pages 4573-4576, XP055651153,
- BURTON D J ET AL: "Preparation, stability and acidity of difluoromethylene bis phosphonic acid", JOURNAL OF FLUORINE CHEMISTRY, ELSEVIER, NL, vol. 20, no. 5, 1 July 1982 (1982-07-01), pages 617-626, XP028086723, ISSN: 0022-1139, DOI: 10.1016/S0022-1139(00)82287-3 [retrieved on 1982-07-01]
- CHARLES E. MCKENNA ET AL: "SYNTHESIS OF [alpha]-HALOGENATED METHANEDIPHOSPHONATES 1a, b", PHOSPHORUS AND SULFUR AND THE RELATED ELEMENTS., vol. 37, no. 1-2, 1 May 1988 (1988-05-01), pages 1-12, XP055650780, CH ISSN: 0308-664X, DOI: 10.1080/03086648808074346
- MCKENNA, C. E. ET AL.: 'Synthetic approaches to biologically active bisphosphonates and phosphonocarboxylates' PHOSPHORUS, SULFUR AND SILICON AND THE RELATED ELEMENTS, [Online] vol. 144, no. 1, 17 March 2008, pages 313 - 316, XP008002440 Retrieved from the Internet: <URL:https://doi.org/10.1080/10426509908546 244>
- MCKENNA, C. E. ET AL.: 'Synthesis of a-halogenated methanediphosphonates' PHOSPHORUS AND SULFUR AND THE RELATED ELEMENTS, [Online] vol. 37, no. 1-2, 01 January 1988, pages 1 - 12, XP055463436 Retrieved from the Internet: <URL:https://www.tandfonline.com/doi/abs/10 .1080/03086648808074346>
- OERTELL, K. ET AL.: 'Transition state in DNA polymerase beta catalysis: rate-limiting chemistry altered by base-pair configuration' BIOCHEMISTRY, [Online] vol. 53, no. 11, 03 March 2014, pages 1842 - 1848, XP055449744 Retrieved from the Internet: <URL:DOI: 10.1021/bi500101z>
- TALANIAN, R. V. ET AL.: 'Carbonyldiphosphonate, a Selective Inhibitor of Mammalian DNA Polymerase delta' XP055463440
- SUCATO, C. A. ET AL.: 'DNA Polymerase beta Fidelity: Halomethylene-Modified Leaving Groups in Pre-Steady-State Kinetic Analysis Reveal Differences at the Chemical Transition State' BIOCHEMISTRY, [Online] vol. 47, no. 3, 28 December 2007, pages 870 - 879, XP055449760 Retrieved from the Internet: <URL:DOI: 10.1021/bi7014162>
- ELISA PALMA ET AL: "Bisphosphonates as radionuclide carriers for imaging or systemic therapy", MOLECULAR BIOSYSTEMS, vol. 7, no. 11, 30 August 2011 (2011-08-30), pages 2950-2966, XP055732909, GB ISSN: 1742-206X, DOI: 10.1039/c1mb05242j

## Description

### CROSS-REFERENCE TO RELATED APPLICATIONS

This application claims the benefit of U.S. Provisional Patent Application Nos. 62/304,895, filed on March 7, 2016, and 62/346,391, filed on June 6, 2016.

### BACKGROUND

### FIELD OF THE INVENTION

The invention relates to [¹⁸F]-labeled bisphosphonates and uses thereof.

### RELATED ART

Molecular imaging seeks to visualize, characterize and quantify biological processes in living subjects at the molecular and cellular level (1). In the realm of biomedicine, molecular imaging provides unique tools for the diagnosis and treatment of human diseases, and is an important resource for the development of personalized medicine (2). Two molecular imaging modalities, positron emission tomography (PET) and single-photon emission computed tomography (SPECT) are utilized in clinical settings. Before a PET or SPECT scan, a molecular probe labeled with a radionuclide is injected into the living subject (3). When the radionuclide decays, the resulting radiation can be imaged using detectors surrounding the subject to precisely locate the source of the decay event. While the basic principles of PET are similar to those of SPECT, PET generally has better sensitivity and spatial resolution than SPECT, and provides the possibility of more accurate attenuation correction (4). Among radioisotopes currently exploited for PET imaging, ¹⁸F (Emax 635 keV, t_{1/2} 109.8 min) is attractive for routine PET imaging because of its advantageous chemical and nuclear properties (5). [¹⁸F]-Fluorodeoxyglucose ([¹⁸F]-FDG), a standard radiotracer used for PET neuroimaging and cancer patient management, is used in clinical studies (6). However, [¹⁸F]-FDG is not a highly specific radiotracer. For example, [¹⁸F]-FDG cannot differentiate well between tumor cells and cells with an increased metabolism related to other etiologies, such as infection or inflammation (7), and is not specific for bone. In general, organofluorine chemistry may present challenges in the context of ¹⁸F labelling, which requires a short time scale for the total synthesis (< 4 h) and facile procedures for preparation of precursors and target compounds (8). The development of new target-specific PET probes by exploring novel ¹⁸F radiochemistry is therefore of great importance.

Bisphosphonates (BPs) bind avidly to bone mineral and are potent inhibitors of osteoclast-mediated bone resorption (9). Increasing evidence from preclinical studies and clinical trials demonstrate that BPs not only act on osteoclasts but also on other cell types including tumor cells (10,11). Although the cellular targets and molecular mechanism of BPs have not yet been fully elucidated, recent data present evidence that BPs can act on tumor cells outside the skeleton by binding to areas of small, granular microcalcifications engulfed by tumor-associated macrophages (12).

BPs are also significant in radiolabeled imaging agents. SPECT imaging with ^{99m}Tc-labeled BPs (e.g., ^{99m}Tc-methylene diphosphonate [^{99m}Tc-MDP] and ^{99m}Tc-hydroxymethyene diphosphonate [^{99m}Tc-HMDP]) remains one of the most common imaging procedures for a variety of bone disorders (13). However, [^{99m}Tc]-MDP and [^{99m}Tc]-HMDP have not been fully optimized from a chemical and pharmaceutical perspective, given some ambiguity about their chemical compositions or structures *in vivo* (14-16) ELISA PALMA et al., Molecular Biosystems, 2011, 7, 2950-2966, disclose bisphosphonates as radionuclide carriers for imaging or systemic therapy.

Importantly, global shortages of technetium-99m emerged in the late 2000s because the two nuclear reactors (NRU and HFR) that provided about two-thirds of the world's supply of molybdenum-99 (precursor of ^{99m}Tc), were shut down repeatedly for extended maintenance periods (17). Even should these supply and also reactor product security-related issues be addressed in the future, it is known that SPECT scanning with [^{99m}Tc]-labeled BPs can have disadvantages for medical imaging, such as relatively low sensitivity and specificity, long uptake and long scan times. In the search for alternative, improved imaging approaches, attention has recently been focused on Na¹⁸F for bone PET scans (18). Because PET imaging with Na¹⁸F is likely to be an uncertain tool for deciphering the molecular mechanisms of BPs and accurate assessment of response to treatment with antiresorptive BPs, a novel ¹⁸F radiochemistry to directly and rapidly radiolabel BPs, combining the advantages of [¹⁸F]-PET imaging with the chemical and pharmacological definition of non-metal complexing BP is desirable.

### SUMMARY

Embodiments of the present invention provide a novel method for rapidly and efficiently introducing fluorine into the P-C-P backbone of bisphosphonates starting from readily preparable diazomethylenebisphosphonate esters. This method has been successfully applied to [¹⁸F]-labeling of bisphosphonates for positron emission tomography imaging.

In one aspect, a method of preparing a fluorinated bisphosphonate, including a fluorine-labeled bisphosphonate, is provided, allowing for rapid introduction of the fluorine atom under conditions suitable for radiochemical labeling of the bisphosphonate with [¹⁸F]. Some embodiments include reacting a diazomethylenebisphosphonate tetraalkyl ester with an HF/base complex and a salt of HF in the presence of a *t*-butyl hypohalite to produce a halofluoromethyl enebisphosphonate tetraalkyl ester, and dealkylating the halofluoromethyl enebisphosphonate alkyl ester to produce a halofluoromethylenebis(phosphonic acid).

The method includes reacting a compound of the formula (I) 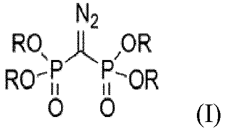 with a fluorinating agent in the presence of an acidic HF/base complex and a *t*-butyl hypohalite (*t*-BuOX) to produce a compound of the formula (II), 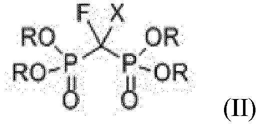 and dealkylating the compound of the formula (II) to produce a
halofluoromethylenebis(phosphonic acid) of the formula (III) 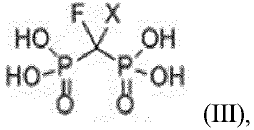 where X is halogen, and each R is the same or different and is independently alkyl or benzyl, and the fluorinating agent is H¹⁸F or a salt thereof, and F of the formulas (II) and (III) is ¹⁸F.

In the method: a) X can be Cl or Br; b) the alkyl can be a C₁-C₃ alkyl; c) each R can be the same; d) the fluorinating agent is H¹⁸F, or a salt thereof, where the salt can be, but is not limited to, K¹⁸F, Na¹⁸F, Cs¹⁸F, Bu₄N¹⁸F or Et₄N¹⁸F; e) the base in the acidic HF/base complex can be, but is not limited to, pyridine, triethylamine or 1,3-dimethyl-3,4,5,6-tetrahydro-2(1H)-pyrimidinone (DMPU); f) a molar ratio of the compound of formula (I):HF can be in the range of about 1:0.05 to about 1:4; g) the temperature of the reaction mixture can be in the range of about - 20 °C to about +20 °C; h) the *t*-butyl hypohalite can be *t*-butyl hypochlorite or *t*-butyl hypobromite; i) the dealkylation can be carried out by treatment with bromotrimethylsilane while heating, followed by hydrolysis with water or an alcohol such as, but not limited to, methanol or ethanol, with some embodiments further comprising microwave irradiation during the dealkylation; j) the halofluoromethylenebis(phosphonic acid) can be selected from the group consisting of 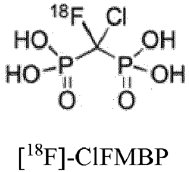 and 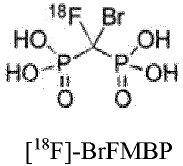 and k) any suitable combination of a) - j) may be used.

Alternatively, in some embodiments, with suitable modification of the reaction conditions, the dealkylation step with bromotrimethylsilane can precede the fluorination step to produce an intermediate tetrakis(trimethylsilyl) ester of the compound of formula (I), where R is trimethylsilyl. This intermediate can be reacted with the fluorinating agent as described above, to produce the halofluoromethylenebis(phosphonic acid). The method includes dealkylating a compound of the formula (I) 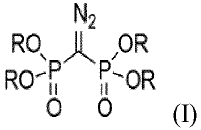 by treatment with bromotrimethylsilane to produce an intermediate tetrakis(trimethylsilyl) ester of the compound of formula (I), and reacting the intermediate with the fluorinating agent in the presence of the acidic HF/base complex and the *t*-butyl hypohalite (*t*-BuOX) to produce a compound of the formula (III) 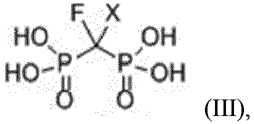 where X is halogen and each R is the same or different and is independently alkyl or benzyl and wherein the fluorinating agent is H¹⁸F or a salt thereof, and F of the formula (III) is ¹⁸F.

In embodiments of this alternate method, a) X can be Cl or Br; b) the fluorinating agent is H¹⁸F, or a salt thereof, where the salt can be, but is not limited to, K¹⁸F, Na¹⁸F, Cs¹⁸F, Bu₄N¹⁸F or Et₄N¹⁸F; c) the F of the formula (III) is ¹⁸F; d) the base in the acidic HF/base complex can be, but is not limited to, pyridine, triethylamine or 1,3-dimethyl-3,4,5,6-tetrahydro-2(1H)-pyrimidinone (DMPU); e) a molar ratio of the intermediate:HF can be in the range of about 1:0.05 to about 1:4; f) the temperature of the reaction mixture can be in the range of about - 20 °C to about +20 °C; g) the *t*-butyl hypohalite can be *t*-butyl hypochlorite or *t*-butyl hypobromite; h) the compound of the formula (III) can be selected from the group consisting of 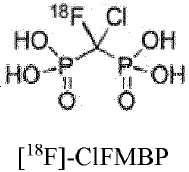 and 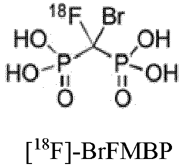 and i) any suitable combination of a) - h) may be used.

In another aspect, a method of preparing a fluoromethylenebis(phosphonic acid) is provided. The method includes treating a compound of the formula (IIIa) 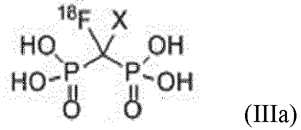 with a reducing agent to produce a fluoromethylenebis(phosphonic acid) of the formula (Va),

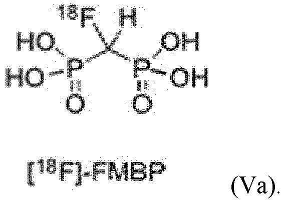

X is Cl or Br.

In a further aspect, another method of preparing a fluoromethylenebis(phosphonic acid) is provided. The method includes dehalogenating a compound of the formula (IIa) 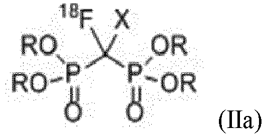 with a reducing agent to produce a compound of the formula (IVa), 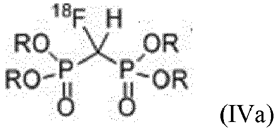 and dealkylating the compound of the formula (IVa) to a fluoromethylenebis(phosphonic acid) of the formula (Va), 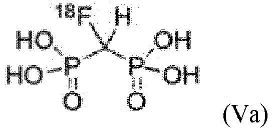 wherein X is Cl or Br, and each R is the same or different and is independently alkyl or benzyl.

In some embodiments: a) the alkyl can be a C₁-C₃ alkyl; b) each R can be the same; or c) any combination of a) - b).

In another aspect, an ¹⁸F-labeled bisphosphonate, or a salt thereof, prepared by any of the methods described herein, are provided. In some embodiments, the bisphosphonate can be any fluorinated bisphosphonate, or a salt thereof, shown in Schemes 1-4. In some embodiments, the bisphosphonate can be a compound, selected from the group consisting of 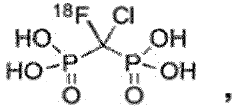 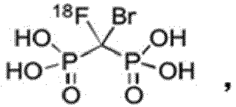 or 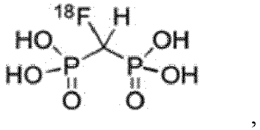 or a salt thereof. The salt is a physiologically acceptable salt or a pharmaceutically acceptable salt. Pharmaceutical compositions including one or any combination of the ¹⁸F-labeled bisphosphonates, or pharmaceutically acceptable salts thereof, are provided along with a carrier. The carrier can be a pharmaceutically acceptable carrier.

In a further aspect, a method *of in vivo* positron emission tomography (PET) imaging is provided. The method includes injecting a subject with an aqueous solution comprising an ¹⁸F-labeled bisphosphonate prepared by any of the methods described herein, or a physiologically acceptable or pharmaceutically acceptable salt thereof, and acquiring a PET scan of the subject by detecting the injected ¹⁸F-label. In the method, the subject can be a human or an animal. In some embodiments, the bisphosphonate can be one or any combination of the fluorinated bisphosphonates [¹⁸F]-ClFMBP, [¹⁸F]-BrFMBP or [¹⁸F]-FMBP, or a physiologically acceptable or pharmaceutically acceptable salt thereof.

In further embodiments, compound [¹⁸F]-ClFMBP is modified by replacing the Cl atom from the compound with an H atom to give bisphosphonate [¹⁸F]-FMBP. This replacement will strengthen the basicity of the bisphosphonate PO-groups, leading to greater bone affinity. The pharmacokinetics and potential toxicity of the compounds will also be somewhat different from the Cl-containing compounds.

In further embodiments, the same replacement is effected starting with [¹⁸F]-BrFMBP in lieu of the corresponding chloro-bisphosphonates.

In further embodiments, the same replacement is effected starting with an alkyl, C₁-C₃ alkyl or benzyl ester of the Cl- or Br- precursor bisphosphonate [¹⁸F]-ClFMBP (or [¹⁸F]-BrFMBP), followed by conversion of the resulting [¹⁸F]-FMBP alkyl, C₁-C₃ alkyl or benzyl ester to the corresponding [¹⁸F]-FMBP product by one of the methods described herein.

### BRIEF DESCRIPTION OF THE DRAWINGS

For a more complete understanding of the present invention, reference is now made to the following descriptions taken in conjunction with the accompanying drawings, in which:
Figure 1 is an analytical HPLC profile of crude compound 7a.
Figure 2 is a semi-preparative HPLC UV (top) and radioactivity (bottom) profile for. [¹⁸F]-ClFMBP.
Figure 3 is a panel showing results after [¹⁸F]-ClFMBP injection. (3A) MicroPET images of a mouse at 2 hours post-injection. (3B) MicroPET quantification of major organs at 2 hours post-injection.

### DETAILED DESCRIPTION

A common approach to the synthesis of α-fluorinated bisphosphonates is electrophilic fluorination of the corresponding carbanions using *N*-fluoro reagents such as Selectfluor. [¹⁸F]-Selectfluor bis(triflate) has been prepared recently using high specific activity ¹⁸F-F₂ (19); however, [¹⁸F]-Selectfluor has not been yet widely adopted for [¹⁸F]-labeling due to the non-trivial requirement for an electrical discharge chamber (20). More generally, electrophilic fluorination of bisphosphonates is conventionally slow and cumbersome in the context of [¹⁸F]-syntheses, where total synthesis time is restricted by the short t_{1/2} of the radioisotope. Recently, Emer et al. reported an efficient nucleophilic ¹⁸F-fluorination of 1-(diazo-2,2,2-trifluoroethyl)arenes with ¹⁸F-labeled Olah reagent (21). However, the inventors' attempts to apply this procedure to several diazomethylenebisphosphonate esters were unsuccessful, possibly due to the lower reactivity of neutral diazo BPs (22). With a view to satisfying the [¹⁸F]-labeling desiderata of simplicity, rapidity and efficiently high yields, reaction of a diazomethylenebisphosphonate alkyl ester in the presence of *t*-butyl hypochlorite (*t*-BuOCl) (23) with F⁻ was considered. HF or an equivalent source of HF can provide both the labelling atom and a Brønsted acid to activate the *t*-BuOCl reagent. Olah's reagent (HF pyridine) offers a safe and convenient source of HF. The inventors succeeded in fluorinating diazo BPs using Olah reagent in the presence of *t*-BuOCl, resulting in the introduction of one chlorine atom and one fluorine atom. Based on the chloro compounds, this approach can be adapted to introduce one bromine atom and fluorine atom.

Scheme 1 describes the synthesis of halofluoromethylenebis(phosphonic acids) **(6a, 6b)** from diazomethylenebisphosphonate alkyl esters (e.g., **2** or **3)** by the new method.

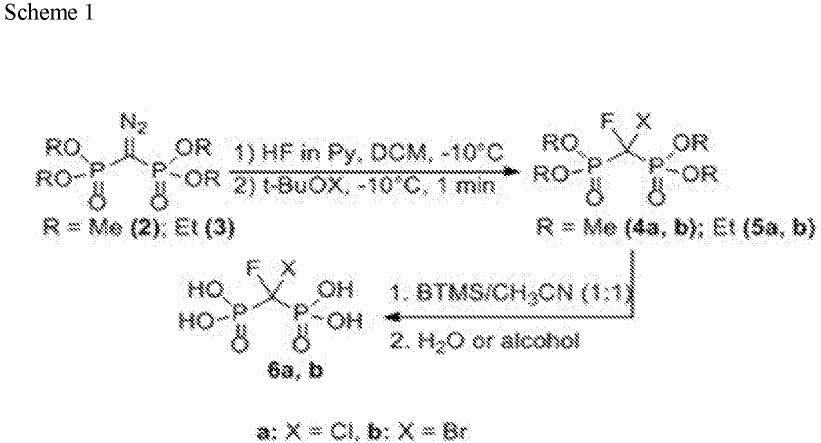

In embodiments containing chloride compounds, diazomethylenebisphosphonate tetramethyl **(2)** and tetraethyl **(3)** esters, prepared according to the literature (24), were placed in a polypropylene tube with formulation of the corresponding solution of Olah reagent in dichloromethane (DCM). A slight excess of *t*-BuOCl (2.5 Eq) was added to the reaction mixture at -10°C. Upon warming to room temperature, the reaction proceeded with rapid evolution of N₂, and formation of chlorofluoromethylenebisphosphonate **(4a, 5a)** in 87% and 82% yield, respectively by ³¹P NMR and MS. A minor side product was identified as the dichloromethylenebisphosphonate ester (10-12%). The demethylation **of 4a** was easily accomplished by brief (15 min) reaction with bromotrimethylsilane (BTMS) (25) in acetonitrile at 80 °C followed by instantaneous conversion to the tetraacid by contact with water (or an alcohol) to afford chlorofluoromethylenebis(phosphonic acid) **6a** in quantitative yield (Scheme 1). BTMS de-ethylation of **5a** was also completed in 20 min, assisted by microwave irradiation at 60 °C. Overall, the preparation of **6a** was achieved in two fast and convenient steps from readily available starting materials.

Scheme 2 describes the synthesis of [¹⁸F]-ClFMBP **1a** and [¹⁸F]-BrFMBP **1b** by the novel method of radiolabeling diazomethylenebisphosphonate esters.

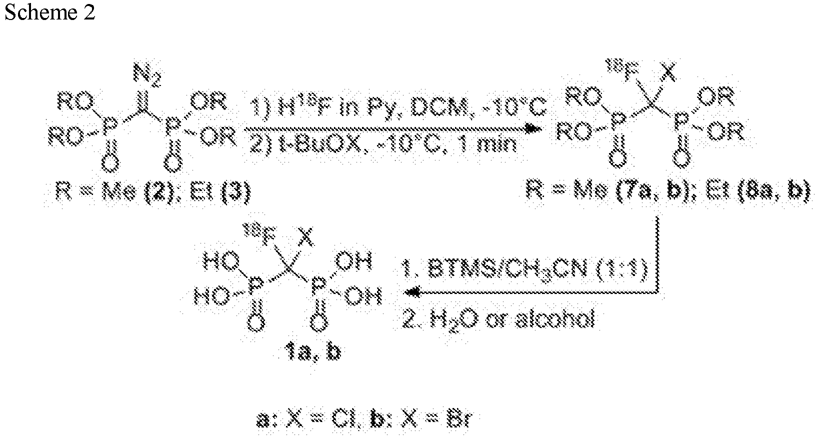

The [¹⁸F]-labeling of tetraethyl and tetramethyl bisphosphonate esters can be carried out according to the method under various conditions. For example, [¹⁸F]-poly(hydrogen fluoride)pyridinium (H¹⁸F/Py), prepared according to a previously reported procedure (26), was used in [¹⁸F] radiofluorinations of bisphosphonate esters and the intermediate product was analyzed by analytical HPLC. When tetraethyl bisphosphonate ester (**3**, 7 mg) was mixed with H¹⁸F/Py (10 µL) and *t*-butyl hypochlorite (15 µL), the radiofluorination was completed within 1 min with cessation of N₂ evolution. The desired tetraethyl chloro[¹⁸F]-fluoromethylenebisphosphonate **8a** was formed in 56% radiochemical yield (RCY), which was not improved by using greater excess of the reagents. As BTMS dealkylation of the tetraethyl ester required a longer heating time (or microwave irradiation assistance) (27,28), radiofluorination of tetramethyl bisphosphonate ester **2** was found to be advantageous. Excess of H¹⁸F/Py decreased the RCY, which may be due to the instability of tetramethyl diazomethylenebisphosphonate **2** in the presence of excess HF reagent. Tetramethyl diazomethylenebisphosphonate **(2,** 5.5 mg), H¹⁸F/Py (15 µL), and *t*-BuOCl (15 µL) provided **7a** with the highest RCY (55.3%). After semi-preparative HPLC purification, **7a** was obtained in 45 ± 8% RCY (decay-corrected, n = 3).

Demethylation of **7a** followed the conditions established for the ¹⁹F-containing tetramethyl ester **4a.** A mixture of **7a** in acetonitrile and BTMS (1:1 ratio) was heated for 15 min at 80 °C affording after hydrolysis [¹⁸F]-ClFMBP **(1a).** The radiochemical purity of [¹⁸F]-ClFMBP was determined to be >99%. The specific activity of the final product was estimated to be 11.7 mCi/µmol.

Based on the chloride compounds, these methods can be adapted to prepare unlabeled and [¹⁸F]-labeled BrFMBP compounds, e.g. **7b, 8b** and **1b.**

Scheme 3 describes the synthesis of [¹⁸F]-FMBP by reduction of [¹⁸F]-ClFMBP or [¹⁸F]-BrFMBP.

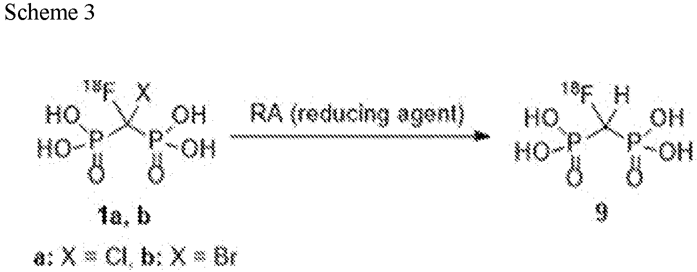

Compounds **1a** or **1b** can be used to synthesize [¹⁸F]-FMBP (compound **9)** by replacing the chlorine or bromine atom in either starting compound by a hydrogen atom. This replacement can be effected rapidly by use of a suitable reducing agent (RA) under appropriate conditions, as shown in Scheme 3. An example of such a reducing agent might be excess aqueous sodium dithionite applied at a temperature between room temperature and 90 °C for a period of less than 30 min. Examples of other reducing agents include, but are not limited to, SnCl₂ or NaHSO₃ (30), or H₂/Pd/C or H₂/PtO₂.

Alternatively, Scheme 4 describes the synthesis of [¹⁸F]-FMBP by selective dehalogenation of [¹⁸F]-ClFMBP or [¹⁸F]-BrFMBP alkyl or benzyl esters, followed by dealkylation.

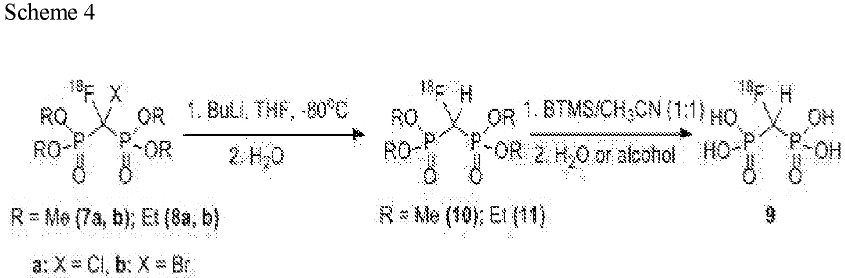

In this alternative method, compound **9** can be synthesized in two steps, beginning with a tetraalkyl ester of [¹⁸F]-ClFMBP or [¹⁸F]-BrFMBP, as shown in Scheme 4 (methyl or ethyl esters **7** or **8** are illustrated, however any alkyl group may be used, particularly any C1-C3 alkyl, or benzyl group). In this approach, the ester may be advantageously dissolved in an organic solvent, e.g. THF or acetonitrile. Selective dehalogenation of the starting ester may be effected by a suitable reducing agent, such as dithionite in a mixed aqueous-organic solvent system with or without a phase transfer catalyst at or somewhat above room temperature for less than 30 min, or alternatively by treatment with 1 :1 or a slight excess of a salt of a carbon compound, e.g. butyl lithium as shown in Scheme 4, at low temperatures in an organic solvent such as THF, for a brief period not exceeding 5 min. Other ways of dehalogenation include, but are not limited to hydrogenolysis catalyzed by Pd or PtO₂. The resulting [¹⁸F]-MBP alkyl ester (such as **10** or **11** in Scheme 4) can then be readily dealkylated to form [¹⁸F]-MBP or a salt thereof, using a method already provided herein, as in Scheme 4.

A fluorine-labeled bisphosphonate can be prepared as a salt, which may be a physiologically acceptable salt or a pharmaceutically acceptable salt. Physiologically acceptable salts and pharmaceutically acceptable salts are well known in the art. Salts formed with, for example, a POH group, can be derived from inorganic bases including, but not limited to, sodium, potassium, ammonium, calcium or ferric hydroxides, and organic bases including, but not limited to, isopropylamine, trimethylamine, histidine, and procaine.

In embodiments involving imaging, the composition may comprise an effective amount of a fluorine-labeled bisphosphonate, or a salt thereof, which can be a physiologically acceptable or pharmaceutically acceptable salt thereof. An effective amount of a compound is an amount that gives emission signals sufficient for PET imaging. As is known, the amount will vary depending on such particulars as the condition of the target tissue, the particular bisphosphonate utilized, and the characteristics of the patient.

Physiologically acceptable carriers and/or diluents, and pharmaceutically acceptable carriers and/or diluents, are familiar to those skilled in the art. For compositions formulated as liquid solutions, acceptable carriers and/or diluents include saline and sterile water, and may optionally include antioxidants, buffers, bacteriostats and other common additives. One skilled in this art may further formulate the compound in an appropriate manner, and in accordance with accepted practices, such as those disclosed in Remington's Pharmaceutical Sciences, Gennaro, Ed., Mack Publishing Co., Easton, Pa. 1990.

Liquid pharmaceutically administrable compositions may, for example, be prepared by dissolving, dispersing, etc., an active compound as described herein and optional pharmaceutical adjuvants in an excipient, such as, for example, water, saline, aqueous dextrose, glycerol, ethanol, and the like, to thereby form a solution or suspension. If desired, the pharmaceutical composition to be administered may also contain minor amounts of nontoxic auxiliary substances such as wetting or emulsifying agents, pH buffering agents and the like, for example, sodium acetate, sorbitan mono-laurate, triethanolamine acetate, triethanolamine oleate, etc. Actual methods of preparing such dosage forms are known, or will be apparent, to those skilled in this art.

The present invention may be better understood by referring to the accompanying examples, which are intended for illustration purposes only and should not in any sense be construed as limiting the scope of the invention.

### EXAMPLE 1

### General Materials and Methods

All the solvents were removed under vacuum at 2 torr. ³¹P NMR and ¹⁹F NMR were recorded on a VNMRS-500 MHz instrument using external D₂O as locking solvent and the ³¹P NMR and ¹⁹F NMR chemical shifts were corrected using 85% phosphoric acid in D₂O (δ 0.00) and hexafluorobenzene (δ -164.9) respectively. Data for ³¹P NMR and ¹⁹F NMR are recorded as follows: chemical shift (δ, ppm), multiplicity (s = singlet, d = doublet, t = triplet). Mass spectrometry (MS) was performed on a Finnigan LCQ Deca XP Max low resolution mass spectrometer equipped with an ESI source in the negative ion mode.

### Cold Chemistry

### Preparation of starting materials

Diazomethylenebisphosphonates **(2, 3)** were prepared according to literature.²⁴ *t*-Butyl hypochlorite was prepared according to the previously reported procedure.²⁹ HF in pyridine and bromotrimethylsilane (BTMS) were directly purchased from Aldrich. BTMS was distilled under nitrogen. Dry DCM and acetonitrile were directly purchased from VWR (drisolv).

### General procedure for the preparation of 4 or 5

**2** or **3** (320 mmol) was dissolved in 0.5 ml of dry DCM in a polypropylene Eppendorf tube. 37 µL of a solution of HF in pyridine (4 eq) was added and the mixture was cooled down to -10°C. 100 µL of t-butyl hypochlorite (2.5 eq) was added. After slight warming to room temperature, the reaction proceeded rapidly with evolution of nitrogen. After the evolution of nitrogen stopped (1 min), the solution was washed with 1 mL of saturated sodium carbonate solution and then washed with water (2×2 ml) and dried over 300 mg anhydrous sodium sulfate and the solvent was removed under vacuum and used for the next reaction without further purification.
Yield: compound **4a,** 87% (by ³¹P NMR). ³¹P NMR (202 MHz, D₂O) δ 9.86, 7.37 (d, *J* = 74.1 Hz); ¹⁹F NMR (470 MHz, CDCl₃) δ -144.16 (t, *J* = 75.3 Hz).
Yield: compound **5a,** 82% (by ³¹P NMR). ³¹P NMR (202 MHz, D₂O) δ 8.06, 5.56 (d, *J* = 78.2 Hz); ¹⁹F NMR (470 MHz, CDCl₃) δ -146.91 (t, *J* = 74.4 Hz).

### General procedure for the preparation of 6a

The product residue **4a** or **5a** was dissolved in 0.2 mL dry acetonitrile and freshly distilled BTMS (Aldrich 97% stabilized by silver, 200 µL, (24 eq) was added and the reaction was set to reflux. Dealkylation was completed at 80° C after 15 min. The solvent was then removed by evaporation under vacuum and the residue was treated with methanol, giving after removal of the solvent under vacuum, the product acid **6a** in quantitative yield. ¹⁹F NMR (470 MHz, D₂O) δ -145.48 (t, *J* = 76.9 Hz). MS calcd for CH₃ClFO₆P₂⁻: 226.91 (100.0%), 228.91 (32.0%), [M-H]⁻, found: 227.32 (100.0%), 229.35 (32.0%), MS calcd for CH₃Cl₂O₆P₂⁻: 242.88 (100.0%), 244.88 (63.9%), [M-H]⁻, found: 243.10 (100.0%), 245.30 (64.0%).

### Radiochemistry Experiment

All chemicals were purchased in analytical grade and used without further purification. Analytical reversed-phase high performance liquid chromatography (HPLC) with a Phenomenex Luna C18 reversed phase column (250 × 4.6 mm, 5 micron) was performed on a Dionex UltiMate 3000 system (Thermo Fisher Scientific, Inc.). The flow was 1 mL/min, with the mobile phase starting from 100% solvent A (0.1% TFA in water) for 5 min, followed by a gradient mobile phase to 20% solvent A and 80% solvent B (0.1% TFA in acetonitrile) at 6 min and isocratic mobile phase with 80% solvent B until 15 min. The UV absorbance was monitored at 254 nm. The radioactivity was detected by a model of Ludlum 2200 single-channel radiation detector. Semi-preparative reversed phase HPLC with a Phenomenex Luna C18 reversed phase column (250 × 10 mm, 5 µm) was carried out on a Knauer BlueShadow Integrated LPG System (Bay Scientific, Inc.). The flow rate was 4 mL/min, with the mobile phase starting from 100% solvent A (0.1% TFA in water) for 7 min, followed by a gradient mobile phase to 20% solvent A and 80% solvent B (0.1% TFA in acetonitrile) at 8 min and isocratic mobile phase with 80% solvent B until 18 min. The UV absorbance was monitored at 254 nm. The radioactivity was detected by a solid-state radiation detector (Carroll & Ramsey Associates).

### Radiochemistry

The radiolabeling reactions were carried out using the following protocol unless otherwise specified.

### Radiosynthesis of [¹⁸F]-poly(hydrogen fluoride)pyridinium.

Cyclotron-produced [¹⁸F] fluoride ion (0.74-1.85 GBq) in [¹⁸O] water was passed through a pre-conditioned QMA cartridge (ABX GmbH, Germany). After removal of [¹⁸O] water, the retained [¹⁸F]fluoride was eluted with an aqueous solution of K₂CO₃ (2.3 mg in 400 µL). The solution was then evaporated to remove water and provide anhydrous [¹⁸F]-KF. Then, 15 µL of (HF)ₙ pyridinium was added, and the solution was incubated at room temperature for 15 min so that the radioactivity can be incorporated into the perfluorinating agent. The solution was used for the next step without further purification.

### Radiosynthesis of tetramethyl (chloro [¹⁸ F]-fluoromethylene)bisphosphonate (7a).

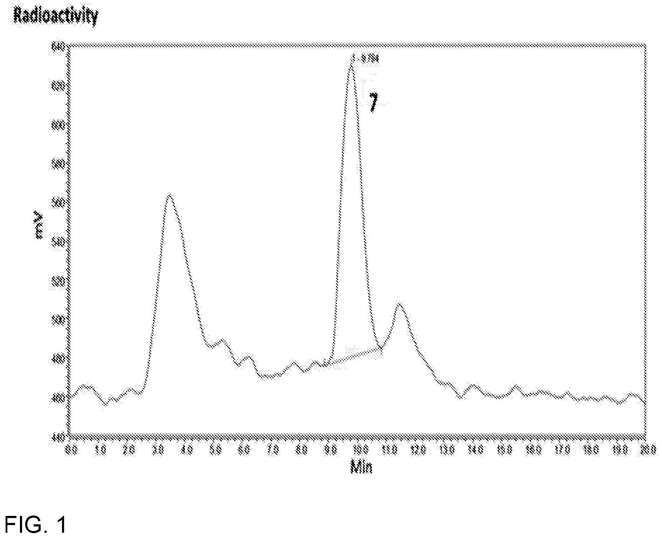

To an Eppendorf tube containing tetramethyl diazomethylenebisphosphonate (5.5 mg) dissolved in 50 µL of dry dichloromethane, [¹⁸F]-poly(hydrogen fluoride)pyridinium (15 µL) was added. The mixture was then cooled to -10°C using dry ice, and 15 µL of t-butyl hypochlorite was added into the mixture. After the evolution of nitrogen stopped (< 1 min), the solution was evaporated under reduced pressure. The residue was re-dissolved in 20% acetonitrile in water, and analyzed by analytical HPLC. The radiofluorinated **7a** was eluted out at 9.78 min. The HPLC result of crude **7a** is shown in Fig. 1. The radiofluorinated **7a** was purified by semi-preparative HPLC and eluted out at 13.2 min.

### Radiosynthesis of (chloro[¹⁸F]-fluoromethylene)bisphosphonic acid ([¹⁸F]-ClFMBP).

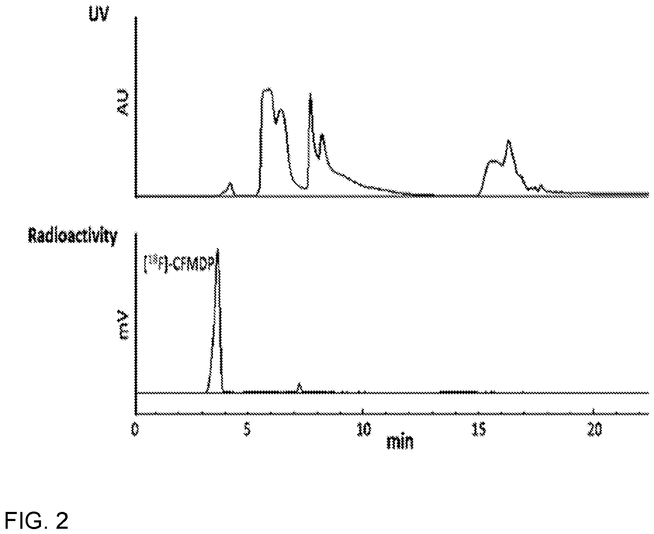

To the solution containing **7a** in 200 µL of acetonitrile, 200 µL of bromotrimethylsilane (BTMS) was added. The mixture was heated at 80°C for 15 min. After the reaction was completed, volatiles were removed by evaporation under vacuum, and 0.5 mL of deionized water was added into the residue. The reaction mixture was then loaded onto semi-preparative HPLC for purification. The HPLC fraction containing [¹⁸F]-ClFMBP (t*_{R}* = 3.6 min) was collected. The HPLC result is shown in Fig. 2. The HPLC eluent was removed using a rotary evaporator. [¹⁸F]-ClFMBP was then reconstituted in 0.9% sodium chloride injection solution and adjusted to pH 7.0. The specific activity of the final product was estimated to be 11.7 mCi/µmol based on 20% conversion of **7a** from [¹⁸F]-poly(hydrogen fluoride)pyridinium. The final product was passed through a 0,22-µm Millipore filter into a sterile vial for small animal study.

### Animals

All animal studies were approved by the University of Southern California Institutional Animal Care and Use Committee. Female athymic nude mice (about 4-6 weeks old, with a body weight of 20-25 g) were obtained from Harlan Laboratories (Livermore, CA). MicroPET scans were performed using an Inveon microPET scanner (Siemens Medical Solutions, Malvern, PA, USA). A normal nude mouse was anesthetized using 2% isoflurane and injected with 1.3-2.5 MBq of [¹⁸F]-ClFMBP via tail vein. At 0.5, 1, and 2 h post injection, static emission scans were acquired for 10 min. Raw PET images were reconstructed using 2D ordered subset expectation maximization (OSEM) algorithms with scatter, random and attenuation correction.

### EXAMPLE 2

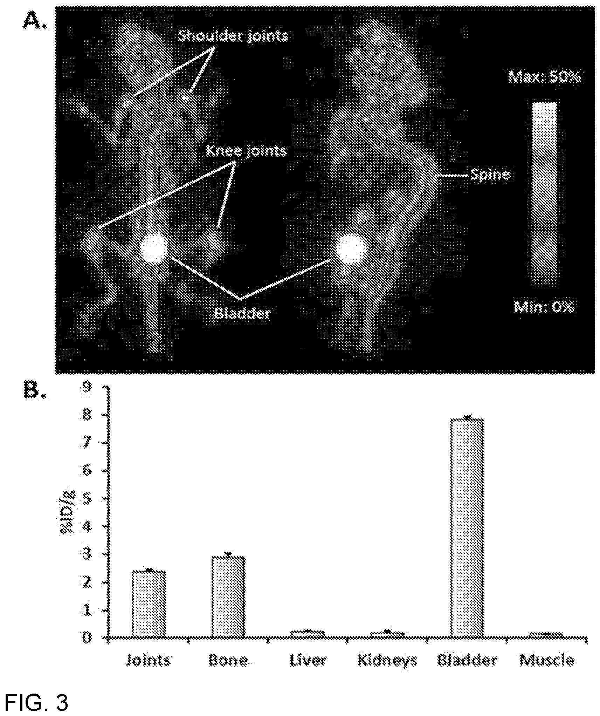

Fig. 3A shows MicroPET images of a mouse at 2 h post-injection of purified [¹⁸F]-ClFMBP. In order to demonstrate its potential for *in vivo* PET imaging, [¹⁸F]-ClFMBP was injected into normal nude mice that were imaged using a microPET scanner at 0.5, 1, and 2 h post-injection. The joints and bones were clearly visible with high contrast to contralateral background at all of imaging time points. The 2D projection of PET images at 2 h post-injection is shown. Predominant uptake of radioactivity was also observed in the bladder, suggesting the excretion of [¹⁸F]-ClFMBP is mainly through the renal system.

Fig. 3B shows MicroPET quantification of major organs at 2 h post-injection of purified [¹⁸F]-ClFMBP. At 2 h post-injection, the uptake of [¹⁸F]-ClFMBP in mouse liver and kidneys was calculated to be 0.21 ± 0.04 and 0.16 ± 0.08 %ID/g (%injected dose per gram of tissue), respectively, which are significantly lower than the values in joints (2.37 ± 0.08 %ID/g) and bones (2.72 ± 0.05 %ID/g). Accumulation of [¹⁸F]-ClFMBP in other mouse organs was minimal.

### REFERENCES

1. R. Weissleder and U. Mahmood, Radiology, 2001, 219, 316-333.
2. K. Chen and P. S. Conti, Adv Drug Deliv Rev, 2010, 62, 1005-1022.
3. S. L. Pimlott and A. Sutherland, Chem Soc Rev, 2011, 40, 149-162.
4. A. Rahmim and H. Zaidi, Nucl Med Commun, 2008, 29, 193-207.
5. P. W. Miller, N. J. Long, R. Vilar and A. D. Gee, Angew Chem Int Ed Engl, 2008, 47, 8998-9033.
6. K. Chen and X. Chen, Semin Oncol, 2011, 38, 70-86.
7. L. Jiang, Y. Tu, H. Shi and Z. Cheng, J Biomed Res, 2014, 28, 435-446.
8. A. P. Wolf, J Fluorine Chem, 1983, 23, 412-412.
9. P. Clezardin, P. Fournier, S. Boissier and O. Peyruchaud, Curr Med Chem, 2003, 10, 173-180.
10. F. Daubine, C. Le Gall, J. Gasser, J. Green and P. Clezardin, J Natl Cancer Inst, 2007, 99, 322-330.
11. P. G. Fournier, F. Daubine, M. W. Lundy, M. J. Rogers, F. H. Ebetino and P. Clezardin, Cancer Res, 2008, 68, 8945-8953.
12. S. Junankar, G. Shay, J. Jurczyluk, N. Ali, J. Down, N. Pocock, A. Parker, A. Nguyen, S. Sun, B. Kashemirov, C. E. McKenna, P. I. Croucher, A. Swarbrick, K. Weilbaecher, T. G. Phan and M. J. Rogers, Cancer Discov, 2015, 5, 35-42.
13. K. K. Wong and M. Piert, J Nucl Med, 2013, 54, 590-599.
14. K. Ogawa and A. Ishizaki, Biomed Res Int, 2015, 2015, 676053.
15. S. Tanabe, J. P. Zodda, E. Deutsch and W. R. Heineman, Int J Appl Radiat Isot, 1983, 34, 1577-1584.
16. L. Qiu, J. G. Lin, X. H. Ju, X. D. Gong and S. N. Luo, Chinese J Chem Phys, 2011, 24, 295-304.
17. T. Ruth, Nature, 2009, 457, 536-537.
18. H. Jadvar, B. Desai and P. S. Conti, Semin Nucl Med, 2015, 45, 58-65.
19. H. Teare, E. G. Robins, A. Kirjavainen, S. Forsback, G. Sandford, O. Solin, S. K. Luthra and V. Gouverneur, Angew Chem Int Ed Engl, 2010, 49, 6821-6824.
20. O. Jacobson, D. O. Kiesewetter and X. Y. Chen, Bioconjugate Chem, 2015, 26, 1-18.
21. E. Emer, J. Twilton, M. Tredwell, S. Calderwood, T. L. Collier, B. Liegault, M. Taillefer and V. GouveNeur, Org Lett, 2014, 16, 6004-6007.
22. C. E. McKenna and J. N. Levy, J Chem Soc Chem Commun, 1989, 246-247.
23. C. E. McKenna and B. A. Kashemirov, in New Aspects in Phosphorus Chemistry I, ed. J.-P. Majoral, Springer Berlin Heidelberg, 2002, vol. 220, pp. 201-238.
24. A. B. Khare and C. E. McKenna, Synthesis-Stuttgart, 1991, 405-406.
25. C. E. McKenna and J. Schmidhauser, J Chem Soc Chem Comm, 1979, 739-739.
26. O. Josse, D. Labar, B. Georges, V. Gregoire and J. Marchand-Brynaert, BioorgMed Chem, 2001, 9, 665-675.
27. A. P. Kadina, B. A. Kashemirov, K. Oertell, V. K. Batra, S. H. Wilson, M. F. Goodman and C. E. McKenna, Org Lett, 2015, 17, 2586-2589.
28. D. A. Mustafa, B. A. Kashemirov and C. E. McKenna, Tetrahedron Lett, 2011, 52, 2285-2287.
29. C. N. Barry and S. A. Evans, J Org Chem, 1983, 48, 2825-2828.
30. C.E. McKenna, L.A. Khawli, W.-Y. Ahmad, P. Pham, and J.-P. Bongartz, Phosphorus and Sulfur and the Related Elements, 1988, 37, 1 - 12.

## Claims

1. A method of preparing a fluorinated bisphosphonate, comprising:
- reacting a compound of the formula (I) 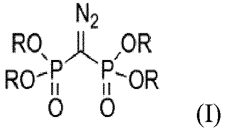 with a fluorinating agent in the presence of an acidic HF/base complex and a *t*-butyl hypohalite (*t*-BuOX) to produce a compound of the formula (II), 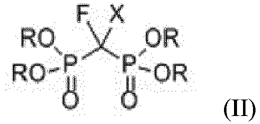
- and dealkylating the compound of the formula (II) to produce a
halofluoromethylenebis(phosphonic acid) of the formula (III), 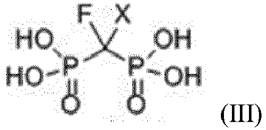 wherein X is halogen, each R is the same or different and is independently alkyl or benzyl, and the fluorinating agent is H¹⁸F or a salt thereof, and F of the formulas (II) and (III) is ¹⁸F.

2. The method of claim 1, wherein
- X is Cl or Br, or
- the alkyl is C₁-C₃ alkyl, or
- each R is the same, or
- the base in the acidic HF/base complex is pyridine, triethylamine or 1,3-dimethyl-3,4,5,6-tetrahydro-2(1H)-pyrimidinone (DMPU), or
- a molar ratio of the compound of the formula (I):HF is in the range of about 1:0.05 to about 1:4, or
- the *t*-butyl hypohalite is *t*-butyl hypochlorite or *t*-butyl hypobromite, or
- the dealkylation is carried out by treatment with bromotrimethylsilane while heating, followed by hydrolysis with water or an alcohol.

3. The method of claim 1, wherein the salt is K¹⁸F, Na¹⁸F, Cs¹⁸F, Bu₄N¹⁸F or Et₄N¹⁸F.

4. The method of claim 1, wherein the dealkylation is carried out by treatment with bromotrimethylsilane while heating, followed by hydrolysis with water or an alcohol and further comprising microwave irradiation during the dealkylation.

5. The method of claim 1, wherein the halofluoromethylenebis(phosphonic acid) is selected from the group consisting of 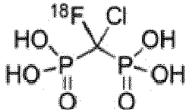 and

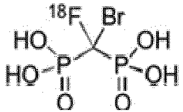

6. A method of preparing a fluoromethylenebis(phosphonic acid), comprising
- treating a compound of the formula (IIIa) 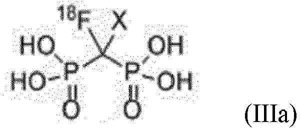 with a reducing agent to produce a fluoromethylenebis(phosphonic acid) of the formula (Va), 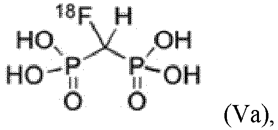 wherein X is Cl or Br.

7. A method of preparing a fluoromethylenebis(phosphonic acid), comprising:
- dehalogenating a compound of the formula (IIa) 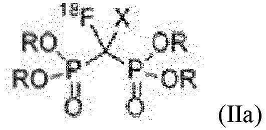 with a reducing agent to produce a compound of the formula (IVa), 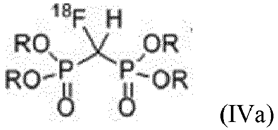 and dealkylating the compound of the formula (IVa) to a fluoromethylenebis(phosphonic acid) of the formula (Va), 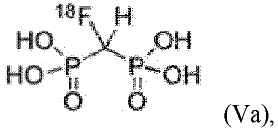 wherein X is Cl or Br, and each R is the same or different and is independently alkyl or benzyl.

8. The method of claim 7, wherein
- the alkyl is a C₁-C₃ alkyl, or
- each R is the same.

9. A method of preparing a fluorinated bisphosphonate, comprising:
- dealkylating a compound of the formula (I) 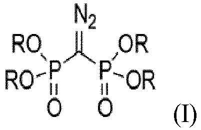 by treatment with bromotrimethylsilane to produce an intermediate tetrakis(trimethylsilyl) ester of the compound of formula (I), and
- reacting the intermediate with a fluorinating agent in the presence of an acidic HF/base complex and a *t*-butyl hypohalite (*t*-BuOX) to produce a compound of the formula (III) 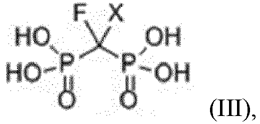 wherein X is halogen and each R is the same or different and is independently alkyl or benzyl and wherein the fluorinating agent is H¹⁸F or a salt thereof, and F of the formula (III) is ¹⁸F.

10. A bisphosphonate compound prepared by the method of claim 1 or claim 9 with the formula 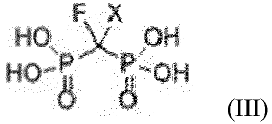 wherein X is halogen and F of the formula (III) is ¹⁸F.

11. A bisphosphonate compound selected from the group consisting of 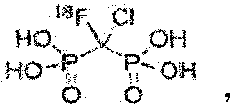 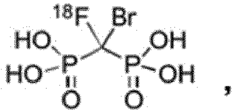 or 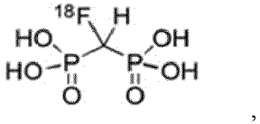 or a pharmaceutically acceptable salt thereof.

12. A pharmaceutical composition comprising one or any combination of the bisphosphonate compounds of claim 11, and a pharmaceutically acceptable carrier.

13. A method of *in vivo* positron emission tomography (PET) imaging, comprising
- injecting a subject with an aqueous solution comprising one or any combination of the ¹⁸F-labeled bisphosphonate compounds of claim 11, and
- acquiring a PET scan of the subject.

## Patentansprüche

1. Verfahren zur Herstellung eines fluorierten Bisphosphonats, umfassend:
- Umsetzen einer Verbindung der Formel (I) 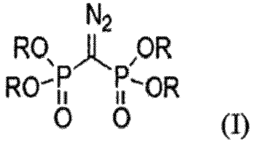 mit einem Fluorierungsmittel in Anwesenheit eines sauren HF/Basen-Komplexes und eines *t*-Butyl-Hypohalits (*t*-BuOX), um eine Verbindung der Formel (II) herzustellen, 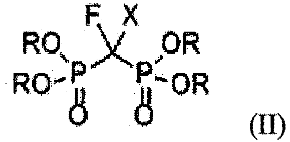
- und Dealkylieren der Verbindung der Formel (II), um eine Halofluormethylen-bis-(phosphonsäure) der Formel (III) herzustellen, 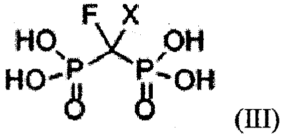 wobei X Halogen ist, jedes R gleich oder verschieden ist und unabhängig Alkyl oder Benzyl ist, und das Fluorierungsmittel H¹⁸F oder ein Salz davon ist, und F der Formeln (II) und (III) ¹⁸F ist.

2. Verfahren nach Anspruch 1, wobei
- X Cl oder B ist, oder
- das Alkyl C₁-C₃-Alkyl ist, oder
- jedes R gleich ist, oder
- die Base in dem sauren HF/Basen-Komplex Pyridin, Triethylamin oder 1,3-Dimethyl-3,4,5,6-tetrahydro-2(1H)-pyrimidinon (DMPU) ist, oder
- ein Molverhältnis der Verbindung der Formel (I) : HF im Bereich von etwa 1:0,05 bis etwa 1:4 liegt, oder
- das *t*-butyl-Hypohalit *t*-butyl-Hypochlorit oder *t*-butyl-Hypobromit ist, oder
- die Desalkylierung durch Behandlung mit Bromtrimethylsilan unter Erhitzung durchgeführt wird, gefolgt von einer Hydrolyse mit Wasser oder einem Alkohol.

3. Verfahren nach Anspruch 1, wobei das Salz K¹⁸F, Na¹⁸F, Cs¹⁸F, Bu₄N¹⁸F oder Et₄N¹⁸F ist.

4. Verfahren nach Anspruch 1, wobei die Desalkylierung durch Behandlung mit Bromtrimethylsilan unter Erhitzung durchgeführt wird, gefolgt von einer Hydrolyse mit Wasser oder einem Alkohol und ferner Mikrowellenbestrahlung während der Desalkylierung umfasst.

5. Verfahren nach Anspruch 1, wobei die Halofluormethylen-bis-(phosphonsäure) ausgewählt ist aus der Gruppe bestehend aus 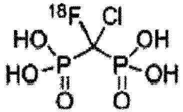 und

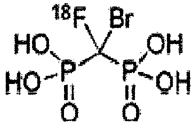

6. Verfahren zur Herstellung einer Fluormethylen-bis(phosphonsäure), umfassend:
- Behandeln einer Verbindung der Formel (IIIa) 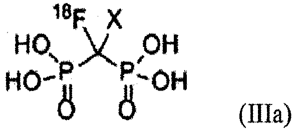 mit einem Reduktionsmittel zur Herstellung eines Fluormethylen-bis(phosphonsäure) der Formel (Va), 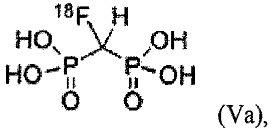 wobei X Cl oder Br ist.

7. Verfahren zur Herstellung einer Fluormethylen-bis(phosphonsäure), umfassend:
- Deshalogenieren einer Verbindung der Formel (IIa) 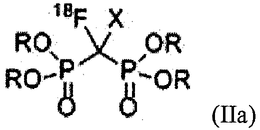 mit einem Reduktionsmittel, um eine Verbindung der Formel (IVa) herzustellen, 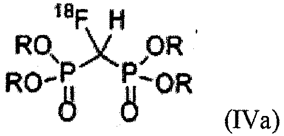 und Desalkylieren der Verbindung der Formel (Iva) zu einer Fluormethylen-bis(phosphonsäure) der Formel (Va), 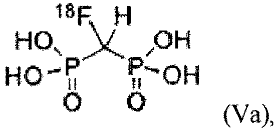 wobei X Cl oder Br ist, und jedes R gleich oder verschieden ist und unabhängig Alkyl oder Benzyl ist.

8. Verfahren nach Anspruch 7, wobei
- das Alkyl ein C₁-C₃-Alkyl ist, oder
- jedes R gleich ist.

9. Verfahren zur Herstellung eines fluorierten Bisphosphonats, umfassend:
- Dealkylieren einer Verbindung der Formel (I) 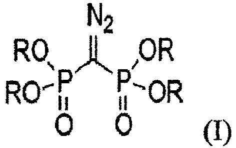 durch Behandlung mit Bromtrimethylsilan zur Herstellung eines Zwischen-Tetrakis(trimethylsilyl)-Esters der Verbindung der Formel (I), und
- Umsetzen des Zwischenprodukts mit einem Fluorierungsmittel in Anwesenheit eines Sauren HF/Basen-Komplexes und eines t-Butyl-Hypohalits (*t*-BuOX), um eine Verbindung der Formel (III) herzustellen 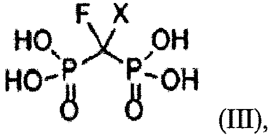 wobei X Halogen ist und jedes R gleich oder verschieden ist und unabhängig Alkyl oder Benzyl ist und wobei das Fluorierungsmittel H¹⁸F oder ein Salz davon ist, und F aus der Formel (III) ¹⁸F ist.

10. Bisphosphonat-Verbindung, hergestellt durch das Verfahren nach Anspruch 1 oder Anspruch 9 mit der Formel 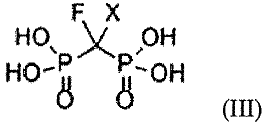 wobei X Halogen ist und F aus der Formel (III) ¹⁸F ist.

11. Bisphosphonat-Verbindung, ausgewählt aus der Gruppe bestehend aus 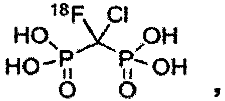 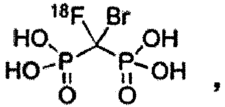 oder 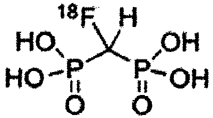 oder eines pharmazeutisch annehmbaren Salzes dieser.

12. Pharmazeutische Zusammensetzung, aufweisend eine oder eine beliebige Kombination der Bisphosphonat-Verbindungen aus Anspruch 11, und einen pharmazeutisch annehmbaren Träger.

13. Verfahren zur *in vivo* Positronen-Emissions-Tomographie (PET), umfassend:
- Injizieren, in eine Testperson, einer wässrigen Lösung, die eine oder eine beliebige Kombination der ¹⁸F-markierten Bisphosphonat-Verbindungen aus Anspruch 11 aufweist, und
- Erlangen eines PET-Scans der Testperson.

## Revendications

1. Procédé de préparation d'un bisphosphonate fluoré, comprenant:
- faire réagir un composé de formule (I) 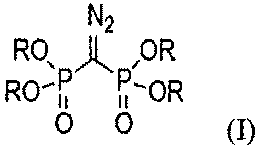 avec un agent de fluoration en présence d'un complexe HF acide/base et d'un hypohalite de *t*-butyle (*t*-BuOX) afin de produire un composé de formule (II), et 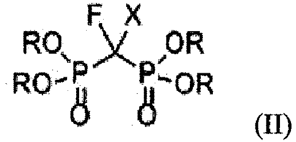
- désalkyler le composé de la formule (II) afin de produire un halofluorométhylènebis (acide phosphonique) de formule (III), 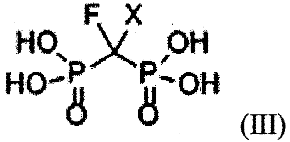 dans lequel X est un halogène ; chaque R est identique ou différent, et est indépendamment alkyle ou benzyle, et l'agent de fluoration est H¹⁸F ou un sel de celui-ci, et F des formules (II) et (III) est ¹⁸F.

2. Procédé selon la revendication 1, dans lequel
- X est Cl ou Br, ou
- l'alkyle est un alkyle en C₁-C₃, ou
- chaque R est identique, ou
- la base dans le complexe HF acide/base est de la pyridine, de la triéthylamine, ou une 1,3-diméthyl-3,4,5,6-tétrahydro-2(1H)-pyrimidinone (DMPU), ou
- un rapport molaire entre composé de formule (I) : HF est compris dans la plage allant d'environ 1 : 0,05 à environ 1 : 4, ou
- le hypohalite de t-butyle est un hypochlorite de t-butyle ou un hypobromite de t-butyle, ou
- la désalkylation est réalisée par un traitement avec un bromotriméthylsilane tout en chauffant, suivi d'une hydrolyse à l'eau ou avec un alcool.

3. Procédé selon la revendication 1, dans lequel le sel est K¹⁸F, Na¹⁸F, Cs¹⁸F, Bu₄N¹⁸F, ou Et₄NK¹⁸F.

4. Procédé selon la revendication 1, dans lequel la désalkylation est réalisée par un traitement au bromotriméthylsilane tout en chauffant, suivi d'une hydrolyse à l'eau ou avec un alcool, et comprenant en outre une irradiation par microondes durant la désalkylation.

5. Procédé selon la revendication 1, dans lequel le halofluorométhylènebis (acide phosphonique) est sélectionné parmi le groupe consistant en 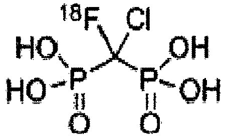 et

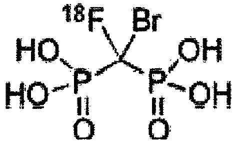

6. Procédé destiné à préparer un fluorométhylènebis (acide phosphonique), comprenant:
- traiter un composé de formule (IIIa) 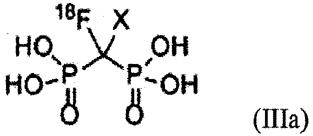 avec un agent de réduction afin de produire un fluorométhylènebis (acide phosphonique) de formule (Va), 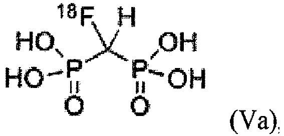 dans lequel X est Cl ou Br.

7. Procédé destiné à préparer un fluorométhylènebis (acide phosphonique), comprenant :
- déshalogéner un composé de formule (IIa) 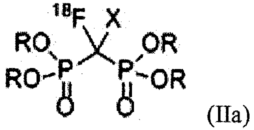 avec un agent de réduction afin de produire un composé de formule (IVa), et 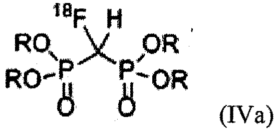
- désalkyler le composé de formule (IVa) en un fluorométhylènebis (acide phosphonique) de formule (Va), 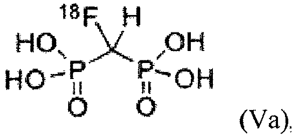 dans lequel X est Cl ou Br, et chaque R est identique ou différent, et est indépendamment alkyle ou benzyle.

8. Procédé selon la revendication 7, dans lequel
- l'alkyle est un alkyle en C₁-C₃, ou
- chaque R est identique.

9. Procédé de préparation d'un bisphosphonate fluoré, comprenant les étapes suivantes :
- désalkyler un composé de formule (I) 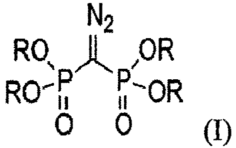 par traitement avec un bromotriméthylsilane afin de produire un ester tétrakis(triméthylsilylique) intermédiaire du composé de formule (I), et
- faire réagir le produit intermédiaire avec un agent de fluoration en présence d'un complexe HF acide/base et d'un hypohalite de t-butyle (t-BuOX) afin de produire un composé de formule (III), 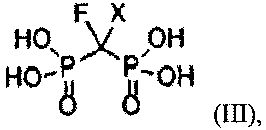 dans lequel X est un halogène, chaque R est identique ou différent, et est indépendamment alkyle ou benzyle, et dans lequel l'agent de fluoration est H¹⁸F ou un sel de celui-ci, et F de la formule (III) est ¹⁸F.

10. Composé de bisphosphonate préparé par le procédé selon la revendication 1 ou la revendication 9 de formule 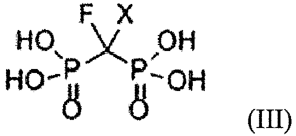 dans lequel X est un halogène, et F de formule (III) est ¹⁸F.

11. Composé de bisphosphonate sélectionné parmi le groupe consistant en : 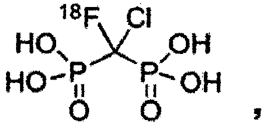 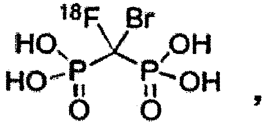 ou 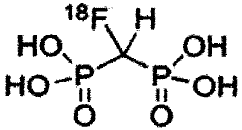 ou un sel de celui-ci acceptable sur le plan pharmaceutique.

12. Composition pharmaceutique comprenant un ou une combinaison quelconque des composés de bisphosphonate selon la revendication 11, et un vecteur acceptable sur le plan pharmaceutique.

13. Procédé destiné à l'imagerie par tomographie par émission de positons (Positron Emission Tomography, PET) *in vivo,* comprenant les étapes d' :
- injecter à un sujet une solution aqueuse comprenant un des composés de bisphosphonate marqués au ¹⁸F ou une combinaison quelconque de ceux-ci selon la revendication 11, et
- acquérir un examen par PET du sujet.
